# EUROPEAN PATENT SPECIFICATION

(11) **EP 4 205 683 B1**
(45) Date of publication and mention of the grant of the patent: **09.12.2026**
(21) Application number: 22217170.4
(22) Date of filing: 29.12.2022
(51) Int. Cl.: A61B 18/14, A61B 5/24, A61B 18/00, A61B 34/20

(54) **COATED MICROELECTRODES**
BESCHICHTETE MIKROELEKTRODEN
MICROELECTRODES REVETUES

(30) Priority: 30.12.2021 US 202117566388
(43) Date of publication of application: 05.07.2023
(73) Proprietor: Biosense Webster (Israel) Ltd, 2066717 Yokneam (IL)
(72) Inventor: GOVARI, Assaf, Yokneam 2066717 (IL); BEECKLER, Christopher Thomas, Irvine 92618 (US)
(74) Representative: Carpmaels & Ransford LLP

(56) References cited:
- EP-A1- 3 106 116
- EP-A1- 3 868 283
- WO-A1-2021/001772
- US-A1- 2017 049 349
- US-A1- 2018 214 202
- US-A1- 2019 059 772
- US-A1- 2019 111 233
- RYLIE A GREEN ET AL: "Substrate dependent stability of conducting polymer coatings on medical electrodes", BIOMATERIALS, ELSEVIER, AMSTERDAM, NL, vol. 33, no. 25, 9 May 2012 (2012-05-09), pages 5875 - 5886, XP028429456, ISSN: 0142-9612, [retrieved on 20120514], DOI: 10.1016/J.BIOMATERIALS.2012.05.017

## Description

### FIELD OF THE DISCLOSURE

The present disclosure is related to the diagnosis and treatment of physiological disorders, such as electrophysiological disorders of a heart.

### BACKGROUND

US Patent 10,874,824 to Beeckler and Keyes, which is assigned to the assignee of the present patent application, describes a method for producing a distal-end assembly of a medical device, the method including disposing a first layer on at least part of a given section among one or more sections of a flexible substrate. At one or more selected locations on the given section, which are to serve as electrodes on the distal-end assembly of the medical device, a second layer is disposed so that, when brought into contact with tissue, the second layer has a reduced impedance for transferring electrical signals to or from the tissue at a predefined frequency range, relative to the first layer. The given section is shaped to form the distal-end assembly.

US Patent 10,939,871 to Altmann et al., which is assigned to the assignee of the present patent application, describes a catheter having a basket-shaped, high density electrode assembly for large-area mapping, and an integrated distal tip providing an array of ultra-high density microelectrodes for acute focal mapping. The basket-shaped electrode assembly has a plurality of electrode-carrying spines (or splines) and the distal tip has a nonmetallic, electrically insulating substrate body with indentations in which microelectrodes are positioned in a manner that the outer surface is generally flush with the outer surface of the substrate body to present a generally smooth, atraumatic distal tip profile.

US Patent Application Publication 2020/0113628 to Govari and Beeckler, which is assigned to the assignee of the present patent application, describes an apparatus having an insertion tube that is configured to be inserted into a body cavity. The apparatus also includes a distal tip connected to the insertion tube, the distal tip having an external surface and a cavity formed in the external surface, the cavity being surrounded by a region of the external surface having a curvature. The apparatus further includes a microelectrode configured to fit into the cavity so that a surface of the microelectrode is contoured, located and oriented to conform with the curvature of the region.

EP3106116A1 discusses an open-irrigated catheter system that comprises a tip section, a distal insert, and mapping electrodes. The tip section has an exterior wall that defines an open interior region within the tip section. The exterior wall includes mapping electrode openings and irrigation ports. The exterior wall is conductive for delivering radio frequency (RF) energy for an RF ablation procedure. The irrigation ports are in fluid communication with the open interior region to allow fluid to flow from the open interior region through the irrigation ports. The distal insert is positioned within the tip section to separate the open region into a distal fluid reservoir and a proximal fluid reservoir. The mapping electrodes are positioned in the mapping electrode openings in the tip section.

Green, R. A et al.("Substrate dependent stability of conducting polymer coatings on medical electrodes", BIOMATERIALS, ELSEVIER, AMSTERDAM, NL, vol. 33, no. 25, 9 May 2012 (2012-05-09), pages 5875-5886, XP028429456, ISSN: 0142-9612, DOI: 10.1016/J.BIOMATERIALS.2012.05.017)is a study into the effects of laser roughening of the underlying platinum electrode surfaces on the mechanical, electrical and biological performance of conducting polymer coatings.

### BRIEF DESCRIPTION OF THE DRAWINGS

The present disclosure will be more fully understood from the following detailed description of examples thereof, taken together with the drawings, in which:
Fig. 1 is a schematic illustration of a system for ablating tissue of a heart of a subject, in accordance with some examples of the present disclosure;
Fig. 2 shows an exploded view of an inset portion of Fig. 1, in accordance with some examples of the present disclosure;
Fig. 3A shows an expanded view of a filled cavity in a tip electrode, in accordance with some examples of the present disclosure;
Fig. 3B shows a cross-section through a filled cavity in a tip electrode, in accordance with some examples of the present disclosure;
Fig. 4 is a schematic illustration of an electrically-insulative rim surrounding a microelectrode, in accordance with some examples of the present disclosure; and
Fig. 5 is a flow diagram for a method for coating microelectrodes, in accordance with some examples of the present disclosure.

### DETAILED DESCRIPTION OF EXAMPLES

### OVERVIEW

The present invention is defined by the appended independent claims. Further embodiments are disclosed by the dependent claims. The present invention refers to ablation probes comprising microelectrodes, which are relatively small sensing electrodes set into the larger tip electrode used for ablation. Given that these microelectrodes must sense electrogram signals having relatively low frequencies at which the impedance at the electrode-tissue interface is relatively high, the outer (tissue-facing) surfaces of the microelectrodes are coated with an impedance-reducing coating.

The coating is a conductive polymer such as poly(3,4-ethylenedioxythiophene) polystyrene sulfonate (PEDOT:PSS), which may be electrochemically polymerized onto the microelectrodes. However, as the probe passes through the delivery sheath used to deliver the probe to the ablation target, the polymer coating may be rubbed off by the inner wall of the sheath.

To address this challenge, some examples of the present disclosure reduce the convexity of the outer microelectrode surfaces relative to the surrounding portions of the tip electrode, such that the likelihood of the polymer coating rubbing against the inner wall of the sheath is reduced. Thus, the outer microelectrode surfaces are concave.

Alternatively or additionally, each microelectrode may be surrounded by an electrically-insulative rim that protrudes beyond the polymeric coating, thereby protecting the coating from the inner wall of the sheath.

### SYSTEM DESCRIPTION

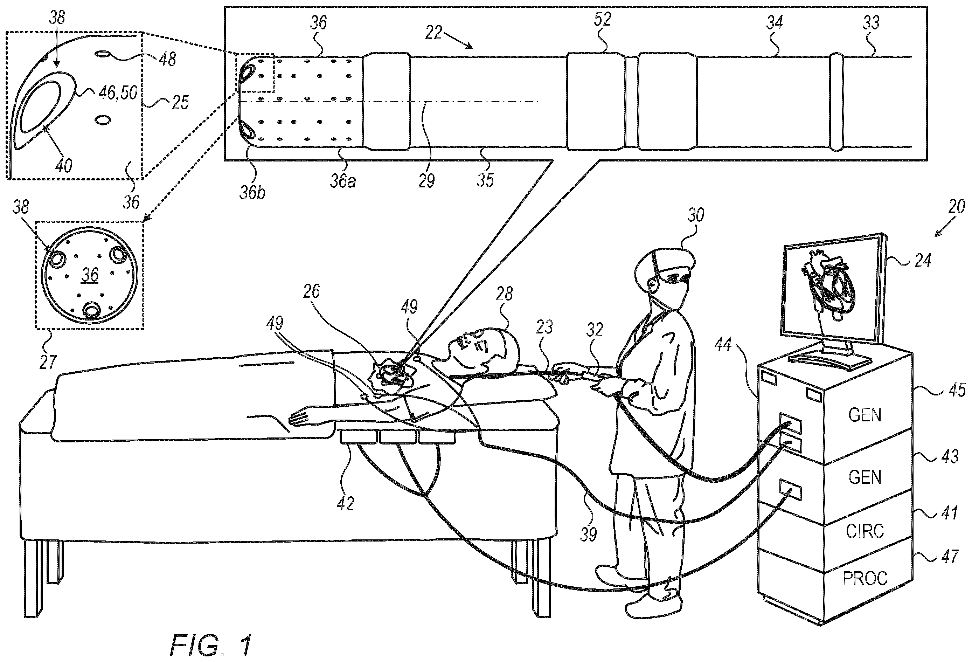

Reference is initially made to Fig. 1, which is a schematic illustration of a system 20 for ablating tissue of a heart 26 of a subject 28, in accordance with some examples of the present disclosure.
34. In some examples, tube 34 comprises multiple segments; for example, the tube may comprise a flexible (or "deflectable") tubular segment 33 distally coupled to one or more coupling elements 35, each of which may be rigid or flexible.

Probe 22 further comprises a tip electrode 36 coupled to the distal end of tube 34, e.g., to the distalmost coupling element 35. Thus, a longitudinal axis 29 of probe 22 extends distally from tube 34 and passes through tip electrode 36.

Tip electrode 36 may be made of gold, platinum, palladium, and/or any other suitable biocompatible metal or metallic alloy. Typically, tip electrode 36 comprises a cylindrical body 36a capped by a dome 36b. Typically, the center of dome 36b is flat, i.e., dome 36b is curved only at its periphery, where the dome meets body 36a.

As shown in an inset portion 25 of Fig. 1, tip electrode 36 is shaped to define one or more (e.g., between two and six) cavities 38, in which respective microelectrodes 40 are disposed. Microelectrodes 40 may be made of gold, platinum, palladium, and/or any other suitable biocompatible metal or metallic alloy.

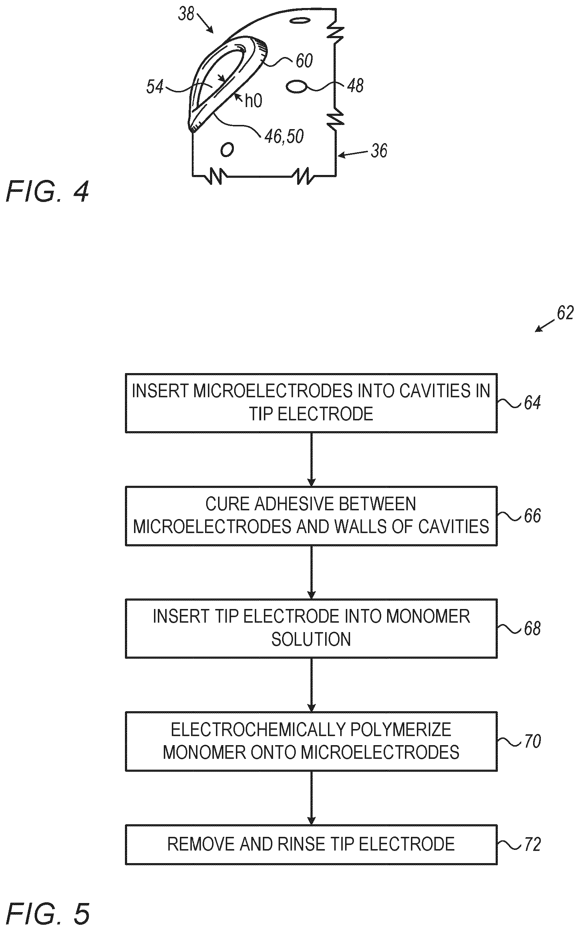

Each microelectrode 40 is insulated from the tip electrode by an electrical insulator 46 that surrounds the microelectrode within cavity 38 such that insulator 46 interposes between the microelectrode and the wall of cavity 38. In some examples, insulator 46 comprises an electrically-insulative polymer 50. For example, as further described below with reference to Fig. 5, the insulator may comprise an adhesive (e.g., an epoxy) that is cured within cavity 38 so as to secure the microelectrode within the cavity. Alternatively or additionally, the insulator may comprise an insert made of an electrically-insulative polymer, ceramic, glass, a combination of any two of the above (e.g., a glass-filled polymer), or any other suitable material. Examples of suitable insulating polymers include polyether ether ketone (PEEK), polycarbonate, and polyethylenimine (PEI).

Typically, cavities 38 (and microelectrodes 40) are distributed around the periphery of dome 36b. For example, as shown in another inset portion 27 of Fig. 1, which shows a frontal view of the tip electrode, three cavities 38, with an angular spacing of approximately 120 degrees, may be distributed around the periphery of dome 36b.

To initiate the ablation procedure, a physician 30 inserts a delivery sheath 23 into the body of subject 28, e.g., via the superior or inferior vena cava of the subject. Subsequently, physician 30, using a control handle 32, navigates the sheath to a chamber of heart 26. Next, the physician deploys probe 22 from the sheath by advancing probe 22 through the sheath, and/or withdrawing the sheath, at least until tip electrode 36 exits the sheath.

System 20 further comprises a signal generator (GEN) 45, which is connected, via wiring running through tube 34, to tip electrode 36 and, via other wiring, to a return electrode coupled to the subject's body. Generator 45 is configured to pass ablation signals between the tip electrode and the return electrode while the tip electrode contacts tissue of heart 26, such that the ablation signals ablate the tissue. Typically, generator 45 is disposed in a console 44, which is coupled to the proximal end of the probe.

In some examples, probe 22 further comprises one or more ring electrodes 52, which may be coupled, for example, to coupling elements 35.

Bipolar electrogram signals from the tissue of heart 26 may be acquired by tip electrode 36 together with any one of microelectrodes 40 or any one of ring electrodes 52. Alternatively or additionally, bipolar electrogram signals may be acquired by a pair of ring electrodes 52, a pair of microelectrodes 40, or a ring electrode together with a microelectrode.

System 20 further comprises circuitry (CIRC) 41, which is disposed in console 44 and comprises a noise filter and an analog-to-digital converter. The aforementioned electrogram signals are carried to circuitry 41 by wiring running through tube 34.

System 20 further comprises a processor 47, which is typically disposed in console 44. Processor 47 is configured to receive the filtered and digitized electrogram signals from circuitry 41 and to process the signals. Processor 47 is further configured to control generator 45.

In some examples, system 20 further comprises a plurality of magnetic-field-generating coils 42 and another signal generator 43. As generator 43 passes electric currents through coils 42, the coils generate a magnetic field. This magnetic field induces signals in electromagnetic sensors coupled to probe 22. The induced signals are carried through tube 34 to circuitry 41. Processor 47 receives the signals from the circuitry and based on the signals, computes the respective locations of the electromagnetic sensors (and hence, of tip electrode 36), e.g., as described in US Patents 5,391,199, 5,443,489, and 6,788,967 to Ben-Haim, in US Patent 6,690,963 to Ben-Haim et al., in US Patent 5,558,091 to Acker et al., and in US Patent 6,177,792 to Govari, whose respective disclosures are incorporated herein by reference.

Alternatively or additionally, system 20 may comprise multiple reference electrodes 49, which may be coupled to the subject's chest and/or back and connected to console 44 via wires running through a cable 39. In such examples, the processor may cause generator 45 to pass a current between any electrode on the probe, such as one of ring electrodes 52, and reference electrodes 49. The processor may then measure the resulting voltages between the probe electrode and reference electrodes 49. Alternatively, the processor may cause generator 45 to apply a voltage between the probe electrode and reference electrodes 49 and measure the resulting currents. Subsequently, the processor may compute the location of the tip electrode based on the measured voltages or currents. Such examples may utilize a location map calibrated using electromagnetic sensors, as described, for example, in US Patent 7,536,218 to Govari et al. and US Patent 8,456,182 to Bar-Tal et al., whose respective disclosures are incorporated herein by reference. (One of reference electrodes 49 may also function as the return electrode for the ablation.)

Alternatively, processor 47 may use any other suitable technique to track the probe while the probe is deployed.

In some examples, probe 22 further comprises a fluid-delivery tube (not shown), which runs through tube 34. The fluid-delivery tube is configured to deliver an irrigating fluid from a pump (not shown), which is typically disposed in console 44, to tip electrode 36. The irrigating fluid passes into the subject's blood via irrigation holes 48 in the tip electrode.

Typically, system 20 further comprises a display 24, configured to display any relevant output. For example, display 24 may display an image or a model of heart 26 with an icon of tip electrode 36 superimposed at the current location of the tip electrode.

Following the procedure, the physician withdraws the probe and/or advances the sheath until the probe enters the sheath. Subsequently, the sheath is withdrawn.

Alternatively to cardiac ablation procedures, probe 22 may be used for any other procedure involving reading signals from and/or passing signals into tissue, including neurological procedures.

### THE MICROELECTRODES

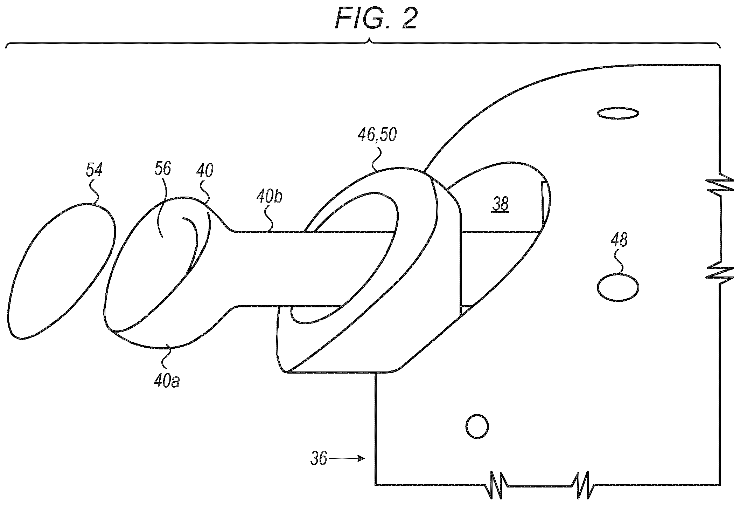

Reference is now made to Fig. 2, which shows an exploded view of inset portion 25 of Fig. 1, in accordance with some examples of the present disclosure. In this Fig. 2, the microelectrode 40 is located near the dome of tip electrode 36 so that temperature of tissue in contact with the tip electrode 36 can be sensed.

A conductive polymeric coating 54, typically comprising poly(3,4-ethylenedioxythiophene) polystyrene sulfonate (PEDOT:PSS), coats the outer surface 56 of each microelectrode.

In some examples, each microelectrode 40 comprises a wider head 40a, which comprises outer surface 56, and a narrower tail 40b. A wire, which passes through tube 34 to console 44 (Fig. 1), is soldered to tail 40b.

Typically, outer surface 56 has an approximately circular perimeter, with a diameter of 0.1 - 0.6 mm, for example.

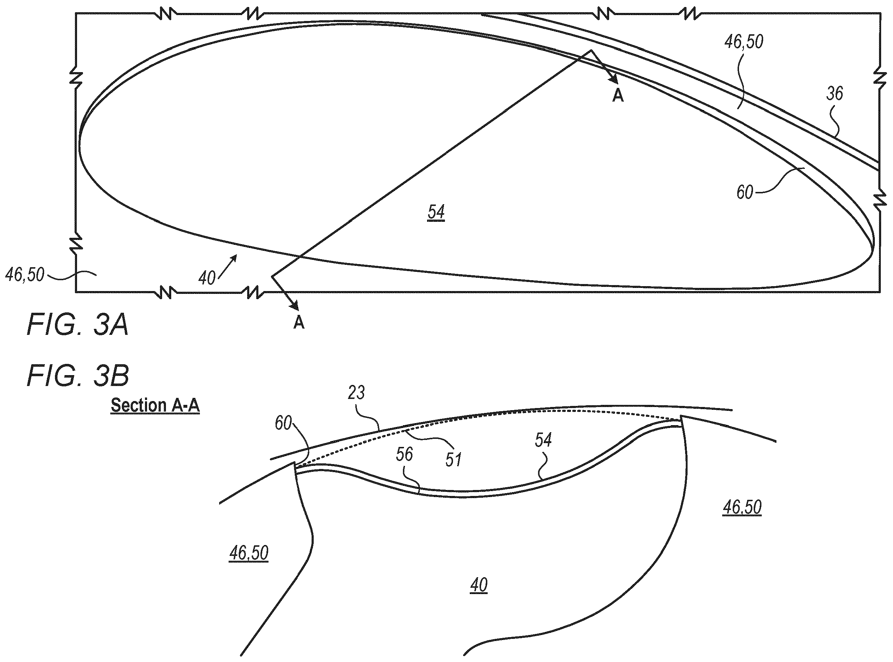

Reference is now made to Fig. 3A, which shows an expanded view of a filled cavity 38, in accordance with some examples of the present disclosure. Reference is also made to Fig. 3B, which shows the cross-section A-A marked in Fig. 3A, in accordance with some examples of the present disclosure.

The outer surface 56 of each microelectrode 40 is of lesser convexity than that of the portion of tip electrode 36 surrounding the cavity 38 in which the microelectrode is disposed. According to the invention shown in Fig. 2 and Figs. 3A-B, outer surface 56 is concave.

By virtue of the lesser convexity of outer surface 56, tip electrode 36 and/or insulator 46 act as a buffer, inhibiting contact between coating 54 and sheath 23 while the probe is passed through the sheath. This advantage is illustrated in Fig. 3B, which shows a hypothetical line 51, which corresponds to the position of coating 54 were the convexity of outer surface 56 approximately the same as that of the surrounding portion of the tip electrode, touching sheath 23.

In some such examples, an electrically-insulative rim 60 surrounds the microelectrode and protrudes beyond coating 54. Rim 60 provides additional protection to the peripheral portions of coating 54, which, typically, are more readily rubbed off than are more central portions of the coating.

For example, insulator 46 may comprise rim 60, i.e., the insulator may surround the microelectrode within the cavity and also protrude from the cavity beyond coating 54. Alternatively, rim 60 may comprise a separate element coupled to insulator 46 and/or to tip electrode 36.

Reference is now made to Fig. 4, which is a schematic illustration of an electrically-insulative rim 60 surrounding a microelectrode, in accordance with some examples of the present disclosure.

In some examples, as illustrated in Fig. 4, the height h0 of rim 60 - defined as the length of the rim in the direction normal to the portion of the surface of the tip electrode abutting the rim - is sufficiently large such that the rim protects coating 54 even if the outer surface of the microelectrode is not of reduced convexity relative to the surrounding portion of tip electrode 36. For example, h0 may be at least approximately 0.1 mm.

Notwithstanding the above, h0 is typically less than approximately 0.5 mm, such that the rim does not inhibit contact between the microelectrode and tissue of the subject. For example, h0 may be between 0.1 and approximately 0.5 mm.

Reference is now made to Fig. 5, which is a flow diagram for a method 62 for coating microelectrodes 40 (Fig. 1), in accordance with an embodiment of the present invention.

Method 62 begins with an inserting step 64, at which the microelectrodes are inserted into the cavities in the tip electrode. Subsequently to the insertion of the microelectrodes, at a curing step 66, an adhesive (e.g., an epoxy) is cured between the microelectrodes and the walls of the cavities, such that the adhesive fastens the microelectrodes to, and also insulates the microelectrodes from, the tip electrode. (The adhesive may be inserted into the cavities before or after the insertion of the microelectrodes.) Alternatively, electrically-insulative inserts may be adhered to the walls of the cavities, and the microelectrodes may then be adhered to the inserts.

As described above with reference to Figs. 2 and Figs. 3A-B, the microelectrodes are of reduced convexity relative to the surrounding portions of the tip electrode. Alternatively or additionally, as described above with reference to Figs. 3A-B and 4, protruding rims may be formed. For example, the rims may be formed during curing step 66, in that the adhesive may be cured so as to define the rims. Alternatively, separate rims may be coupled to the tip electrode and/or to the cured adhesive.

Subsequently, at another inserting step 68, the tip electrode is inserted into a monomer solution, i.e., a solution including a dissolved monomer. The monomer polymerizes into a conductive polymer; for example, the monomer may comprise 3,4-ethylenedioxythiophene (EDOT), which polymerizes into PEDOT:PSS.

(Typically, prior to the insertion of the tip electrode into the solution (e.g., prior to the insertion of the microelectrodes into the tip electrode), the tip electrode is coupled to the distal end of tube 34 (Fig. 1) so as to facilitate the polymerization described immediately below.)

Next, at a polymerizing step 70, the monomer is electrochemically polymerized onto the microelectrodes, such that the microelectrodes become coated with a conductive polymeric coating. For example, EDOT may be polymerized so as to form a coating of PEDOT:PSS.

To perform this polymerization, electric currents are passed between the microelectrodes and a return electrode in the solution, such as a metallic (e.g., platinum) cage surrounding the tip electrode. The durations of the currents are controlled so as to achieve a predetermined coating thickness, which is typically 0.1 - 25 µm (microns).

Finally, at a removing-and-rinsing step 72, the tip electrode is removed from the solution and rinsed.

It will be appreciated by persons skilled in the art that the present disclosure is not limited to what has been particularly shown and described hereinabove. Rather, the scope of the present disclosure includes both combinations and subcombinations of the various features described hereinabove, as well as variations and modifications thereof that are not in the prior art, which would occur to persons skilled in the art upon reading the foregoing description. The invention is defined by the claims.

## Claims

1. An intrabody probe for ablating tissue of a heart(22), comprising:
a tube (34);
a tip electrode (36) coupled to a distal end of the tube (34) and shaped to define at least one cavity (38) in a surface of the tip electrode;
a microelectrode (40) disposed within the cavity (38) and comprising an outer surface (56) that is of lesser convexity than that of a portion of the tip electrode (36) surrounding the cavity (38); and
an impedance-reducing conductive polymeric coating (54) that coats the outer surface (56); and
wherein the outer surface (56) comprises a concave surface such that the likelihood of the polymer coating rubbing against the inner wall of a sheath is reduced; and
the microelectrode is insulated from the tip electrode by an electrical insulator that surrounds the microelectrode within the at least one cavity such that the electrical insulator interposes between the microelectrode and a wall of the cavity;.

2. The intrabody probe (22) according to claim 1, wherein the insulator is an electrically-insulative rim (60) surrounding the microelectrode (40) and protruding beyond the polymeric coating (54).

3. The intrabody probe (22) according to claim 2, further comprising an electrical insulator (46) surrounding the microelectrode (40) within the cavity (38), wherein the electrical insulator (46) comprises the rim (60).

4. A method (62), comprising:
inserting a tip electrode (36), which is configured to couple to a distal end of a tube (34) and is shaped to define a cavity (38) in the surface of a tip electrode, into a solution including a dissolved monomer,
the cavity (38) containing a microelectrode (40) having an outer surface (56) that is of lesser convexity than that of a portion of the tip electrode (36) surrounding the cavity (38);
wherein the microelectrode is insulated from the tip electrode by an electrical insulator that surrounds the microelectrode within the at least one cavity such that the electrical insulator interposes between the microelectode and a wall of the cavity; and
by passing an electric current between the microelectrode (40) and a return electrode in the solution, polymerizing the monomer onto the outer surface (56), thereby coating the outer surface (56) with an impedance-reducing conductive polymeric coating (54);
wherein the outer surface (56) is concave such that the likelihood of the polymer coating rubbing against the inner wall of a sheath is reduced.

5. The method according to claim 4, wherein an electrically-insulative rim surrounds the microelectrode, and wherein coating the microelectrode comprises coating the microelectrode such that the rim protrudes beyond the polymeric coating.

6. The method according to claim 5, wherein an electrical insulator surrounds the microelectrode within the cavity and includes the rim.

7. The apparatus according to any one of claims 1-3, or the method according to claim 4 wherein the conductive polymeric coating (54) includes poly(3,4-ethylenedioxythiophene) polystyrene sulfonate (PEDOT:PSS).

## Patentansprüche

1. Intrakorporale Sonde zum Ablatieren von Gewebe eines Herzens (22), umfassend:
einen Schlauch (34);
eine Spitzenelektrode (36), die an ein distales Ende des Schlauchs (34) gekoppelt und geformt ist, um mindestens einen Hohlraum (38) in einer Oberfläche der Spitzenelektrode zu definieren;
eine Mikroelektrode (40), die innerhalb des Hohlraums (38) angeordnet ist und umfassend eine Außenoberfläche (56), die eine geringere Konvexität vorweist als die eines Abschnitts der Spitzenelektrode (36), der den Hohlraum (38) umgibt; und
eine impedanzreduzierende leitfähige Polymerbeschichtung (54), die die Außenoberfläche (56) beschichtet; und
wobei die Außenoberfläche (56) eine konkave Oberfläche derart umfasst, dass die Wahrscheinlichkeit reduziert wird, dass die Polymerbeschichtung an der Innenwand einer Hülle reibt; und
die Mikroelektrode von der Spitzenelektrode durch einen elektrischen Isolator isoliert ist, der die Mikroelektrode innerhalb des mindestens einen Hohlraums derart umgibt, dass der elektrische Isolator zwischen der Mikroelektrode und einer Wand des Hohlraums angeordnet ist;.

2. Intrakorporale Sonde (22) nach Anspruch 1, wobei der Isolator ein elektrisch isolierender Rand (60) ist, der die Mikroelektrode (40) umgibt und über die Polymerbeschichtung (54) hinweg vorsteht.

3. Intrakorporale Sonde (22) nach Anspruch 2, ferner umfassend einen elektrischen Isolator (46), der die Mikroelektrode (40) innerhalb des Hohlraums (38) umgibt, wobei der elektrische Isolator (46) den Rand (60) umfasst.

4. Verfahren (62), umfassend:
Einführen einer Spitzenelektrode (36), die konfiguriert ist, um an ein distales Ende eines Schlauchs (34) zu koppeln, und geformt ist, um einen Hohlraum (38) in der Oberfläche einer Spitzenelektrode zu definieren, in eine Lösung, die ein gelöstes Monomer einschließt,
wobei der Hohlraum (38) eine Mikroelektrode (40) enthält, die eine Außenoberfläche (56) aufweist, die eine geringere Konvexität vorweist als die eines Abschnitts der Spitzenelektrode (36), der den Hohlraum (38) umgibt;
wobei die Mikroelektrode von der Spitzenelektrode durch einen elektrischen Isolator isoliert ist, der die Mikroelektrode innerhalb des mindestens einen Hohlraums derart umgibt, dass der elektrische Isolator zwischen der Mikroelektrode und einer Wand des Hohlraums angeordnet ist; und
durch Leiten eines elektrischen Stroms zwischen der Mikroelektrode (40) und einer Rückelektrode in der Lösung, Polymerisieren des Monomers auf die Außenoberfläche (56), wodurch die Außenoberfläche (56) mit einer impedanzreduzierenden leitfähigen Polymerbeschichtung (54) beschichtet wird;
wobei die Außenoberfläche (56) derart konkav ist, dass die Wahrscheinlichkeit eines Reibens der Polymerbeschichtung an der Innenwand einer Hülle reduziert wird.

5. Verfahren nach Anspruch 4, wobei ein elektrisch isolierender Rand die Mikroelektrode umgibt, und wobei das Beschichten der Mikroelektrode umfasst, die Mikroelektrode derart zu beschichten, dass der Rand über die Polymerbeschichtung hinweg vorsteht.

6. Verfahren nach Anspruch 5, wobei ein elektrischer Isolator die Mikroelektrode innerhalb des Hohlraums umgibt und den Rand umfasst.

7. Einrichtung nach einem der Ansprüche 1 bis 3 oder Verfahren nach Anspruch 4, wobei die leitfähige Polymerbeschichtung (54) Poly(3,4-ethylendioxythiophen)-Polystyrolsulfonat (PEDOT:PSS) einschließt.

## Revendications

1. Sonde intracorporelle permettant l'ablation de tissu d'un cœur (22), comprenant :
un tube (34) ;
une électrode à pointe (36) couplée à une extrémité distale du tube (34) et profilée pour définir au moins une cavité (38) dans une surface de l'électrode à pointe ;
une microélectrode (40) disposée à l'intérieur de la cavité (38) et comprenant une surface externe (56) qui présente une convexité inférieure à celle d'une partie de l'électrode à pointe (36) entourant la cavité (38) ; et
un revêtement polymère conducteur réduisant l'impédance (54) qui recouvre la surface externe (56) ; et
dans laquelle la surface externe (56) comprend une surface concave de telle sorte que la probabilité que le revêtement polymère frotte contre la paroi interne d'une gaine soit réduite ; et
la microélectrode est isolée de l'électrode à pointe par un isolateur électrique qui entoure la microélectrode à l'intérieur de l'au moins une cavité de telle sorte que l'isolateur électrique s'interpose entre la microélectrode et une paroi de la cavité ;.

2. Sonde intracorporelle (22) selon la revendication 1, dans laquelle l'isolateur est une couronne électriquement isolante (60) entourant la microélectrode (40) et faisant saillie au-delà du revêtement polymère (54).

3. Sonde intracorporelle (22) selon la revendication 2, comprenant en outre un isolateur électrique (46) entourant la microélectrode (40) à l'intérieur de la cavité (38), dans laquelle l'isolateur électrique (46) comprend la couronne (60).

4. Procédé (62), comprenant :
l'insertion d'une électrode à pointe (36), qui est configurée pour se coupler à une extrémité distale d'un tube (34) et est profilée pour définir une cavité (38) dans la surface d'une électrode à pointe, dans une solution comportant un monomère dissous,
la cavité (38) contenant une microélectrode (40) ayant une surface externe (56) qui présente une convexité inférieure à celle d'une partie de l'électrode à pointe (36) entourant la cavité (38) ;
dans laquelle la microélectrode est isolée de l'électrode à pointe par un isolateur électrique qui entoure la microélectrode à l'intérieur de l'au moins une cavité de telle sorte que l'isolateur électrique s'interpose entre la microélectrode et une paroi de la cavité ; et
en faisant passer un courant électrique entre la microélectrode (40) et une électrode de retour dans la solution, en polymérisant le monomère sur la surface externe (56), recouvrant de ce fait la surface externe (56) d'un revêtement polymère conducteur réduisant l'impédance (54) ;
dans laquelle la surface externe (56) est concave de telle sorte que la probabilité que le revêtement polymère frotte contre la paroi interne d'une gaine soit réduite.

5. Procédé selon la revendication 4, dans lequel une couronne électriquement isolante entoure la microélectrode, et dans lequel le revêtement de la microélectrode comprend le revêtement de la microélectrode de telle sorte que la couronne fait saillie au-delà du revêtement polymère.

6. Procédé selon la revendication 5, dans lequel un isolateur électrique entoure la microélectrode à l'intérieur de la cavité et comporte la couronne.

7. Appareil selon l'une quelconque des revendications 1 à 3, ou procédé selon la revendication 4 dans lequel le revêtement polymère conducteur (54) comporte du poly(3,4-éthylènedioxythiophène) polystyrène sulfonate (PEDOT:PSS).
